# EUROPEAN PATENT SPECIFICATION

(11) **EP 2 883 461 B1**
(45) Date of publication and mention of the grant of the patent: **08.11.2017**
(21) Application number: 13197015.4
(22) Date of filing: 12.12.2013
(51) Int. Cl.: A23L 15/00, A61K 38/17, A23B 5/015, A23L 3/015, A23B 5/00, A23L 3/32, A61K 35/57, A23L 33/17, A23B 5/03

(54) **Process for producing active follistatin**
Verfahren zur Herstellung von aktivem Follistatin
Procédé de production de follistatine active

(30) Priority: 11.12.2013 EP 13196770
(43) Date of publication of application: 17.06.2015
(73) Proprietor: Deutsches Institut für Lebensmitteltechnik e.V., 49610 Quakenbrück (DE); OVOBEST Eiprodukte GmbH & Co. KG, 49434 Neuenkirchen-Vörden (DE)
(72) Inventor: Heinz, Volker, 49610 Quakenbrück (DE); Franke, Knut, 49610 Quakenbrück (DE)
(74) Representative: Taruttis, Stefan Georg

(56) References cited:
- EP-A1- 1 714 659
- EP-A1- 2 674 033
- WO-A1-89/01945
- WO-A1-2013/186234
- GB-A- 1 103 683
- US-A1- 2005 287 260
- US-A1- 2007 275 036
- US-A1- 2008 311 259
- D. D'antona ET AL: "Increased maternal serum activin A but not follistatin levels in pregnant women with hypertensive disorders.", Journal of Endocrinology, 2000, pages 157-162, XP055083057, Retrieved from the Internet: URL:http://joe.endocrinology-journals.org/ content/165/1/157.full.pdf [retrieved on 2013-10-08]
- WEI YAN ET AL: "Effect of high pressure treatment on the physicochemical and functional properties of egg yolk", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 231, no. 3, 11 May 2010 (2010-05-11), pages 371-377, XP019839658, ISSN: 1438-2385
- DATABASE WPI Week 200804 Thomson Scientific, London, GB; AN 2008-A49898 XP002685770, & CN 100 998 422 A (UNIV JIANNAN) 18 July 2007 (2007-07-18)
- AMIALI ET AL: "Synergistic effect of temperature and pulsed electric field on inactivation of Escherichia coli O157:H7 and Salmonella enteritidis in liquid egg yolk", JOURNAL OF FOOD ENGINEERING, BARKING, ESSEX, GB, vol. 79, no. 2, 5 April 2006 (2006-04-05), pages 689-694, XP005844979, ISSN: 0260-8774, DOI: 10.1016/J.JFOODENG.2006.02.029
- GÓNGORA-NIETO M M ET AL: "Energy analysis of liquid whole egg pasteurized by pulsed electric fields", JOURNAL OF FOOD ENGINEERING, BARKING, ESSEX, GB; INTERNATIONAL FOOD INFORMATION SERVICE (IFIS), FRANKFURT-MAIN, DE, vol. 57, no. 3, 2003, pages 209-216, XP002685771, ISSN: 0260-8774, DOI: 10.1016/S0260-8774(02)00299-6
- MARTÍN-BELLOSO O ET AL: "Inactivation of Escherichiacoli suspended in liquid egg using pulsed electric fields", JOURNAL OF FOOD PROCESSING AND PRESERVATION, WILEY-BLACKWELL PUBLISHING, INC, TRUMBULL, CT, US; INTERNATIONAL FOOD INFORMATION SERVICE (IFIS), FRANKFURT-MAIN, DE, vol. 21, no. 3, 1997, pages 193-208, XP002685772, ISSN: 0145-8892, DOI: 10.1111/J.1745-4549.1997.TB00776.X [retrieved on 2007-05-05]
- HUANG E ET AL: "Inactivation of Salmonella enteritidis in Liquid Whole Egg using Combination Treatments of Pulsed Electric Field, High Pressure and Ultrasound", BIOSYSTEMS ENGINEERING, ACADEMIC PRESS, UK, vol. 94, no. 3, 15 May 2006 (2006-05-15), pages 403-413, XP024970483, ISSN: 1537-5110, DOI: 10.1016/J.BIOSYSTEMSENG.2006.03.008 [retrieved on 2006-07-01]
- DATABASE FSTA [Online] INTERNATIONAL FOOD INFORMATION SERVICE (IFIS), FRANkFURT-MAIN, DE; 2007, ANDRASSY E ET AL: "Changes of hen eggs and their components caused by non-thermal pasteurizing treatements. II. Some non-microbiological effects of gamma irradiation or hydrostatic pressure processing on liquid egg white and egg yolk.", XP002685773, Database accession no. FS-2007-01-Qa0022 & ACTA ALIMENTARIA, vol. 35, no. 3, 2007, page 305, ACTA ALIMENTARIA 2006 CORRESPONDENCE ADDRESS, J. FARKAS, CENT. FOOD RES. INST., H-1022 BUDAPEST, HUNGARY. FAX +36-1-482 6321. E-MAIL J.FARKAS@CFRI.HU
- WIKIPEDIA ET AL: "Follistatin", INTERNET CITATION, 3 June 2012 (2012-06-03), pages 1-9, XP002714385, Retrieved from the Internet: URL:http://web.archive.org/web/20120603083 205/http://en.wikipedia.org/wiki/Follistat in [retrieved on 2013-10-08]

## Description

The present invention relates to a process for producing a composition comprising biologically active follistatin from a biological source, especially from fertilized avian eggs, preferably egg white, which composition is preserved and, especially pathogen free and is storage stable, preferably at room temperature. Further, the invention relates to the composition containing biologically active follistatin, which composition is available by the production process. Follistatin has been found to be a secreted glycoprotein having activity to inhibit members of the TGF-β family, preferably to inhibit myostatin. Upon ingestion, the composition has activity to support, induce and/or positively regulate the increase of muscle in humans and animals. The composition is therefore suitable for use as a food ingredient or nutrition additive for humans and animals, e.g. for use as a compound for improving muscle increase and/or muscle regeneration.

Preferably, the process for producing the composition, and the composition itself, are free from added chemical preservatives, most preferably, the process for producing the composition, and the composition, respectively, essentially consist of the natural components of the starting material, e.g. egg and its components, preferably egg yolk optionally including the white of egg, only subject to the physical treatment steps of the process. In the alternative to egg yolk, white of egg, which is also called egg white or egg albumen, and whole egg can be subjected to the steps of the process.

### State of the art

US 2007/0275036 A1 describes that follistatin is known to be present in fertilized avian eggs and is biologically active to increase muscle mass and to facilitate muscle regeneration in humans. Whereas the pasteurzation of liquid egg yolk is shown to inactivate the biological activity of follistatin contained therein, it is described that freeze-dried emulsified egg yolk can be irradiated e.g. by gamma radiation or by an electron beam for preservation without inactivating follistatin.

WO89/01945 describes isolation of follistatin from porcine follicular fluid by successive heparin-Sepharose affinity chromatography, gel filtration and several reverse-phase HPLC with different stationary and mobile phases. Using 0.01M NaCl, 0.01 M Tris-HCl, pH 7 for washing and 1M NaCl, 0.01 M Tris-HCl, pH 7 for elution, the Heparin-Sepharose affinity chromatography is described to recover 90% of the activity of the crude porcine follicular fluid.

EP 2674033 and PCT/EP2013/062068, which are relevant under Art. 54(3) EPC only, describe the production of a preserved composition containing follistatin by subjecting whole egg, white of egg, yolk from fertilized avian eggs or raw animal blood serum to a pressure of at least 4500 bar for at least 1 min or to a pulsed electric field of at least 5 kV/cm at a flow rate of 30L/h. Optionally, prior to the preservation, the insoluble egg membranes, i.e. the egg yolk membrane and/or chalazae can be concentrated.

US 2007/0275036 A1 describes freeze-drying of chicken eggs to produce a powder containing active follistatin, whereas gamma-irradiation resulted in no measurable follistatin. WO 89/01945 A1 describes a purification method for follistatin from porcine follicular fluid. EP 1714659 A1 describes the electro-stimulation of a non-human animal and the gamma-irradiation of blood serum obtained from the animal.

US 2005/0287260 A1 describes the preservation of egg products by pasteurizing egg material e.g. at 60 - 65 °C, electromagnetic radiation or high frequency radio waves, followed by pressure treatment and optionally adding an antimicrobial agent.

US 2008/0311259 A1 describes high pressure treatment, especially of liquid egg products for inactivating microorganisms.

D'Antona et al, Journal of Endocrinology 157-162 (2000) describes that follistatin and activin A were analyzed in blood serum samples.

Yan et al, Eur. Foods Res. Technol. 371-377 (2010) relates to the influence of high pressure treatment on the emulsifying activity of egg yolk.

The English abstract of CN100998422 describes that pulsed electric field treatment can be used in preserving liquid egg products in combination with added lysozyme.

Amiali et al, Journal of Food Engineering 689-694 (2007) describes that pulsed electric field treatment of liquid egg yolk can reduce the populations of E. coli and Salmonella enteritidis. Gongora-Nieto et al, Journal of Food Engineering 209-216 (2003) describes that liquid whole egg that was acidified with citric acid was non-thermally preserved by pulsed electric field treatment. Martin-Belloso et al, Journal of Food Processing and Preservation 193-208 (1997) relates to inactivation of E. coli in egg by a pulsed electric field.

Huang et al, Biosystems Engineering 403-413 (2006) describes the inactivation of Salmonella enteritidis in liquid whole egg using pulsed electric field, high pressure and/or ultrasound treatment.

### Objects of the invention

It is an object of the present invention to provide for an alternative process for producing a composition containing active follistatin from fertilized avian eggs or animal blood serum, which process results in a higher concentration of follistatin.

### General description of the invention

The invention achieves the object by the features of the claims, and especially relates to a process for producing a composition containing biologically active follistatin from a starting material, which is selected from fertilized avian eggs or its components, especially from egg yolk, egg white or whole egg, with egg white being the preferred starting material, of fertilized avian eggs, and/or animal blood serum, the process comprising the concentration of follistatin and preservation while maintaining a temperature below 38°C, preferably below 20°C, more preferably below 10°C. Accordingly, the step of concentrating a fraction of soluble protein from the starting material separates a fraction of the soluble protein which contains follistatin, e.g. in a higher concentration in relation to total weight, preferably in relation to total protein of the starting material. The step of concentrating follistatin concentrates follistatin from the soluble or solute protein of the starting material and comprises or consists of separating a soluble protein fraction containing follistatin from the starting material, e.g. egg white. The soluble protein fraction containing follistatin can be separated e.g. by precipitation followed by sedimentation, which preferrably is centrifugation. Precipitation can be caused by acidification, e.g. for egg white to pH 6 or below, e.g. to pH 5.0, more preferred to approx. pH 5.5, and/or by addition of water soluble salt, as a solid or as an aqueous solution and/or addition of a solvent, e.g. methanol, ethanol, and mixtures thereof, to the starting material.

It has been found that the egg white of fertilized hen eggs (Gallus domesticus) has a follistatin concentration of approx. 15 to 30 µg/kg, whereas the egg yolk only contained 1 to 3 µg/kg and approx. 30 % by weight (wt-%) fats. Also in view of its very low to negligible fat content, egg white is the preferred starting material.

Sedimentation of egg white with follistatin precipitated for 24 h at natural gravity was found to result in a concentration of approx. 75 to 80 µg/kg sediment, an increase over egg white in this instance by a factor of 3 to 4. Centrifugation can be made at e.g. at least 800 x g, preferably at least 1000 x g for at least 3 min, preferably at least 5 min.

Precipitation can be by acidification of the starting material, e.g. by addition of acid, e.g. a food-grade acid, especially phosphoric acid, hydrochloric acid, acetic acid, malic acid, citric acid and/or preferably lactic acid, and/or by fermentation of the starting material while reducing the concentration of sugar, especially of the reducing sugar contained in starting material, e.g. in the egg white, by addition of acid producing bacteria, preferably lactic acid producing bacteria, e.g. lactobacilli, e.g. commercially available starter cultures for yoghurt (available from Chr. Hansen A/S, Denmark). Fermentation of egg white can be under conditions known for removal of reducing sugars, e.g. with lactic acid producing bacteria at 4 °C for 18 h. The addidition of acid can be in the form of liquid acid, e.g. as an aqueous solution, or as a solid.

Precipitation can be made by addition of salt, wherein the salt can be a neutral salt, e.g., ammonium acetate and/or NaCl, and/or an acidic salt, e.g. NaHCO₃, and/or a chaotropic salt, e.g. KCl and/or ammonium sulfate. The concentration of the salt or mixture of salts used can be determined by the proportion of follistatin that is contained in the separated precipitate, e.g. determined by SDS-PAGE. For example, for KCl, a concentration of 0.2 M to 0.3 M in egg white can be used, as 0.25 M KCl was found to result in precipitation of a protein fraction containing follistatin that could be separated by centrifugation at 1000 x g for 5 min. When the egg white starting material contains e.g. 16 µg/kg follistatin, the separated fraction was found to contain e.g. 32.4 µg/kg follistatin.

Generally, and especially for low concentrations of follistatin, the follistatin concentration was preferrably determined by an ELISA employing anti-follistatin antibodies, e.g. the Human Follistatin Quantikine ELISA kit available from R&D Systems, Minneapolis, USA, which assay uses antibody raised against recombinantly expressed human follistatin.

Optionally, the precipitated follistatin can be solubilized from the separated precipitate prior to or following preservation, e.g. by an increase of the pH, e.g. to at least 7, preferably at least 8, more preferably to a pH of 8.5 to 9.5, and/or decreasing the salt concentration, e.g. by dialysis or dilution with water.

The step of preservation comprises or consists of subjecting preferably the soluble protein fraction containing follistatin to a pressure of at least 4000 bar, for at least 1 minute, preferably to 5500-6500 bar, more preferably to 6000 bar for at least 1 minute, preferably for 3 minutes, more preferably for at least 5 minutes, preferably using an adiabatic compression and pressure release, and/or pulsed electric field treatment, preferably in a continuous process while pumping the liquid egg or its components, especially egg yolk, egg white or whole egg, through the space limited by at least 2 discharge electrodes, e.g. generating an electric field strength of 5 to 40 kV/cm, e.g. at 5 to 12 kV/cm at a flow rate of the liquid starting material or a separated fraction containing follistatin of 30 L/h at a temperature of 30°C, preferably using unipolar pulses having a pulse duration of 5 to 20µs, preferably of 10µs, at a repetition rate of 70 to 200 Hz, especially positive, rectangular pulses. At an energy input of 50 to 140 kJ/kg, the decrease in bacterial contamination, determined as CFU, was by a factor of 10 to 630, respectively. Preferably, the fertilized avian eggs are obtained from hens, i.e. Gallus domesticus.

The embodiments of the step of preservation are non-thermal process steps, i.e. an increase in temperature that may occur during the high pressure treatment and/or pulsed electric field treatment is not causative for the reduction in micro-organisms, especially of bacteria to achieve preservation. In addition, the embodiments of the step of preservation are physical treatment methods, i.e. without addition of antimicrobial chemical compounds. Accordingly, the embodiments of the step of preservation are non-thermal process steps consisting of physical treatment steps, which do not generate radicals and therefore maintain the chemical structure of the ingredients, especially of unsaturated fatty acids and vitamins of the composition. Accordingly, the composition obtainable by the process preferably contains unsaturated fatty acids in the natural constitution or conformation as in the starting material.

The process has the advantage of the step of concentrating the follistatin rich fraction by precipitation and separation to reduce the volume prior to freeze-drying, which reduces the energy required in this step.

It is a further advantage of the embodiments of the process, in which the follistatin containing protein fraction is separated prior to the step of preservation by high pressure treatment that smaller volumes need high pressure treatment.

The high pressure treatment and/or the pulsed electric field treatment of a starting material, which is liquid whole egg, liquid egg yolk, preferably liquid egg white, and/or animal blood serum, effectively reduces the bacterial contamination by a factor of at least 10, preferably by a factor of at least 100, more preferably of at least 1000. For example, for high pressure treatment, a reduction of the bacterial contamination to about 50 CFU/g, corresponding to a reduction by a factor of 3000 was found when starting from raw liquid egg yolk having a natural bacterial content of 1.5 x 10⁵ CFU/g. For pulsed electric field treatment, a reduction by a factor of 10 to a factor of 1000 was found. The reduction of the natural microbiological contamination by the high pressure treatment and/or the pulsed electric field treatment is sufficient for preserving the egg white, whole egg or egg yolk.

Preferably, the process subsequent to the preservation step comprises drying, especially freeze-drying the follistatin containing soluble protein fraction of the liquid egg, especially egg yolk, egg white or whole egg, or the follistatin containing soluble protein fraction of animal blood serum, respectively, resulting in an egg fraction containing powder, especially an egg yolk, egg white or whole egg containing powder, or animal blood fraction serum powder, e.g. in a powder consisting essentially of the follistatin containing fraction which is high pressure treated and/or pulsed electric field treated. In the alternative to freeze-drying, the drying can be fluidized bed drying, preferably at a temperature at or below 42°C, preferably at or below 40°C, more preferably at or below 38°C or at or below 35°C. Due to drying, the resultant product may comprise water-insoluble protein even if the aqueous fraction mainly contains soluble protein.

It has been found that the process for producing the follistatin - containing composition comprising a preservation step comprising or consisting of high pressure treatment and/or pulsed electric field treatment, preferably with subsequent drying, especially but not limited to freeze-drying, leads both to an efficient reduction of bacterial contamination as determined e.g. as viable bacteria, and to the follistatin maintaining its biological activity, e.g. to at least 50%, preferably to at least 70%, more preferably to at least 80%, more preferably to at least 85%, at least 90% or to at least 95%, with reference to follistatin activity in the starting material.

Especially in view of preservation processes using irradiation, it is an advantage of the process of the invention that no radicals are generated by the step of preservation, and therefore the resulting preserved follistatin containing fraction of liquid egg yolk, egg white or whole egg, or animal blood serum, which preferably is subsequently dried, preferably freeze-dried, contains less or no radicals and reaction products of radicals. E.g. the preserved liquid egg yolk, egg white or whole egg, as well as the dried, preferably freeze-dried, preserved egg yolk, or whole egg, contains unsaturated fatty acids of the egg yolk essentially in their natural state and composition, e.g. without changes to their double bonds. Accordingly, the composition obtainable by the process of the invention preferably contains the unsaturated fatty acids of egg yolk without changes of their double bonds, i.e. in their natural biological constitution.

Preferably, in the process, no chemical preservative is added, e.g. no anti-microbial agent is added. Optionally, an antioxidant is added, e.g. ascorbic acid or a neutral salt thereof. Preferably, the whole egg, egg yolk, more preferably egg white, or animal blood serum, resp., is free from added ingredients, e.g. the whole egg, egg yolk, or more preferably the egg white, or the animal blood serum, resp., is subjected to the physical process steps only, which comprise, preferably consist of separating a follistatin containing soluble protein fraction from the starting material, which is e.g. liquid whole egg, egg white or liquid egg yolk, or animal serum, resp., and subjecting this fraction to high pressure treatment and/or to pulsed electric field treatment, preferably followed by drying, e.g. freeze-drying or fluidized bed drying. In the alternative, the starting material is first subjected to the pulsed electric field treatment and then the follistatin containing fraction is separated from the preserved starting material. For high pressure treatment, it is preferred that the liquid whole egg, white of egg or liquid egg yolk, or animal blood serum, or the follistatin containing fraction thereof, respectively, is contained in sealed containers having an elastic wall, e.g. in plastic bags, more preferably free from gas, more preferably degassed. For a gas-free whole egg, egg white or liquid egg yolk, or animal blood serum, respectively, in a container, gas bubbles can be expelled before sealing the container. For degassing, a reduced pressure can be applied prior to high pressure treatment, preferably also prior to pulsed electric field treatment.

High pressure treatment is generally carried out using water as a compression medium that is pumped into a sealed chamber containing the liquid whole egg, egg white or liquid egg yolk, or animal blood serum, respectively, until the high pressure is reached, maintaining the high pressure, and then releasing the pressure, e.g. by opening the high pressure container.

It was found that after high pressure treatment within sealed containers, e.g. in sealed polyethylene bags, the starting material selected from liquid whole egg, egg white or liquid egg yolk preparation or serum, or the follistatin containing fraction that is separated from the starting material, respectively, is stable, e.g. for 12 to 24 hours, preferably for 2 to 5 days, e.g. at 5 to 10°C, without a drastic increase in bacterial contamination, and especially without a significant loss of follistatin activity.

For high pressure treatment, the adiabatic increase in temperature due to the high pressure preferably is counteracted by cooling the liquid whole egg, egg white or liquid egg yolk or serum, or the follistatin containing fraction separated from the starting material, respectively, to a temperature which is at least 5°C, preferably about 10°C below the maximum temperature, e.g. below 38°C prior to the treatment. Preferably, prior to high pressure treatment and/or prior to the pulsed electric field treatment, the liquid whole egg, egg white or liquid egg yolk or serum, or the follistatin containing fraction separated from the starting material, respectively, is cooled to a temperature of between 0 and 28°C, preferably to 5 to 20°C, more preferably to a maximum of 10°C.

For pulsed electric field treatment, it was found that a short rise in temperature, e.g. to a maximum of 45°C, preferably to a maximum of 42°C or to 40°C, for maximally 10s, preferably for maximally 5 or maximally 2s results in a low loss of active follistatin. Accordingly, for the pulsed electric field treatment, the aforementioned short rise in temperature is acceptable, although less preferred.

Active follistatin was determined by size separation, e.g. by size-exclusion HPLC or by SDS-polyacrylamide gel electrophoresis (SDS-PAGE), optionally followed by Western blotting and immunospecific detection using an anti-follistatin antibody. A reduction of the size-specific signal identified for follistatin in fresh yolk from fertilized eggs was used as an indicator for the reduction of follistatin activity, because an inactivation of follistatin results in the change, e.g. reduction of the molecule size.

Optionally, the process comprises a step of concentrating also the membranes of the whole egg, egg white or egg yolk of the fertilized eggs prior to or concurrent to separating to the follistatin containing fraction. For concentrating the membranes, the fraction of whole egg, of egg white or of egg yolk having the higher proportion of follistatin is used, the fraction being obtained e.g. by size separation or by density separation. The preferred fraction is the fraction containing the egg yolk membrane, e.g. obtained from separating egg yolk or whole egg, and the fraction containing chalazae, e.g. obtained from separating the white of egg or whole egg. The preferred fraction contains the major portion of the egg yolk membranes and/or of the chalazae of the whole egg, egg white or egg yolk subjected to the concentrating step, e.g. precititation and separating step. For separating and concentrating the membranes by size separation, sieving can be used, e.g. of a mesh size of 0.5 mm to 2 mm, preferably approx. 0.5 to 1 mm. Using size separation, the preferred membrane fraction is the egg yolk membrane and/or chalazae containing fraction, which is the particulate or large fraction. For separating by density separation, centrifugation, e.g. using a centrifugal separator. Using density separation of whole egg, egg white or egg yolk, the higher density fraction is the preferred fraction.

Optionally, prior to the step of concentrating the membranes from the whole egg, egg white or egg yolk of the fertilized eggs by separating the fraction containing the egg yolk membrane and/or chalazae, the whole egg, egg white or egg yolk can be diluted to facilitate the separating step, e.g. using water as a diluent, the water optionally containing salt. Further optionally, a salt and/or acid and/or alcohol for precipitation of the water soluble protein fraction containing follistatin can be added, or an acidifying fermentation of the egg white can be made, and the chalazae and/or egg yolk membrane can be separated from the starting material concurrent with the separation of the precipitated soluble protein fraction containing follistatin.

In the alternative or in addition to whole egg, egg white or egg yolk of fertilized eggs, the process can be performed using blood serum from slaughtered animals as the starting material. Accordingly, the blood serum can replace the whole egg, egg white or egg yolk in the process, and therefore the description relating to whole egg, egg white or egg yolk also refers to the process with blood serum as the starting material. In this embodiment, the process for producing a composition containing biologically active follistatin comprises the step of providing raw animal blood serum, concentrating a follistatin containing soluble protein fraction and, prior to or preferably subsequently to concentrating the follistatin containing protein fraction, subjecting the serum to a step of preservation while maintaining the temperature at or below 38°C, wherein the step of preservation is selected from subjecting the serum or the fraction to a high pressure treatment of at least 4500 bar for at least 1 min, from subjecting the serum or fraction to a pulsed electric field treatment of at least 5 kV/cm at a flow rate of 30 L/h, and from a combination of the high pressure treatment and the pulsed electric field treatment to provide a preserved liquid serum fraction containing follistatin. The step of preservation can consist of high pressure treatment, wherein the serum or the fraction containing follistatin subsequent to separation from the starting material is filled into containers, the containers are sealed and subjected to the high pressure. The step of preservation can consist of pulsed electric field treatment of the raw animal blood serum or of the fraction containing follistatin subsequent to separation from the starting material by an electric field of 5 to 40kV/cm. Subsequent to the step of preservation, the preserved serum or fraction, resp., can optinally be dried, e.g. by freeze-drying or by fluidized bed drying. Preferably, between the step of high pressure treatment and the step of freeze-drying, the serum or fraction, resp., is transported in the sealed containers in which the raw animal blood serum was subjected to the high pressure treatment.

Optionally, the process can comprise the further step of mixing or encapsulating the dried preserved follistatin containing fraction of the starting material. Preferably, for mixing or encapsulating, the dried and preserved and follistatin containing fraction of the starting material, is admixed with a solution, preferably an aqueous solution of an encapsulating agent. The encapsulating agent can e.g. be a sugar, sugar alcohol and/or sugar polymer, a solution of which in the process is admixed with the preserved and dried and follistatin containing fraction, and dried to produce encapsulated dried egg yolk, whole egg or egg white. The sugar can e.g. be sucrose, fructose, glucose, and/or corn sirup. The sugar alcohol can e.g. be maltitol, isomalt etc. The sugar polymer can e.g. be starch, modified starch and/or cellulose and/or methylcellulose, which preferably also serves as an anti-caking agent.

As a specific advantage of the high pressure treatment of liquid egg yolk, whole egg or egg white, or serum and of the follistatin containing fraction of the starting material, it has been found that the bioavailability and digestability of the protein is enhanced, while the follistatin essentially maintains its biological activity. Therefore, the process comprising the step of high pressure treatment of the starting material or of the follistatin containing fraction thereof is preferred for producing a preserved composition containing biologically active follistatin, in which composition the protein has increased bioavailability, e.g. increased digestability, for example in relation to the non-treated liquid egg yolk, whole egg or egg white.

In order to produce egg white, whole egg or egg yolk, liquid or dried, containing a desired concentration of active follistatin, e.g. the natural concentration of follistatin of egg white, whole egg or egg yolk from fertilized eggs, the preserved follistatin containing fraction that is separated from the starting material, without drying or subsequent to drying, can be admixed with egg white, whole egg or egg yolk, liquid or dried, obtained from non-fertilized eggs. This step of admixing egg white, whole egg or egg yolk, liquid or dried, obtained from non-fertilized eggs allows that the main portion of the resultant mixture, i.e. the egg white, whole egg or egg yolk, liquid or dried, obtained from non-fertilized eggs can be produced using standard preservation processes, e.g. thermal preservation and/or drying at elevated temperatures.

### Detailed description of the invention

The invention is now described by way of non-limiting examples.

### Example 1: production of a preserved egg white containing active follistatin

Fertilized hen eggs (Gallus domesticus) contained from a certified breeding station were used, which eggs were not brooded. The eggs were cracked and separated into egg yolk and the white of egg automatically. As the starting material, 3000 L egg white were used that were preferably homogenized by stirring were maintained at 5 to 10°C and filled under sterile conditions into polyethylene bags and sealed after expulsion of entrapped air bubbles. These polyethylene bags could have a volume of between 1 L and 50 L, preferably of 5 to 20 L each. The bags were arranged in a high pressure chamber (NC-Hyperbaric, Spain). Using water as a pressurising medium, the pressure was increased to 6000 bar within 10 to 20 minutes. After a holding time of 3 or 5 minutes, respectively, the pressure was released by opening a release valve.

The bacterial contamination was determined by standard dilution plating on complete medium and counting following cultivation in an incubator at 37°C for 48 h.

The microbiological analysis showed that the high pressure treatment both for 3 minutes and 5 minutes resulted in a drastic reduction of bacterial contamination, and also the subsequent step of freeze-drying further reduced the bacterial contamination.

**Table 1: bacterial contamination, measured as CFU/g**

| sample | Salmonella in 25g sample | Total cell count (CFU/g) |
|---|---|---|
| raw liquid egg yolk | Negative | 1.5 x 10⁵ |
| liquid egg yolk after 6000 bar, 3 min | Negative | 50 |
| liquid egg yolk after 6000 bar, 5 min | Negative | 50 |
| freeze-dried egg yolk after 6000 bar, 3 min | Negative | 40 |
| freeze-dried egg yolk after 6000 bar, 5 min | Negative | <10 |

| | | |
|---|---|---|
| CFU = colony forming units (viable micro-organisms) | | |

A follistatin containing fraction was separated from the high pressure treated egg by adding aqueous 12 M lactic acid until the mixture was at pH 5.5 white while maintaining about 5°C. The precipitate, which was visible, was sedimented by centrifugation at 1000 x g for 5 min and formed the follistatin containing fraction, the supernatant was discarded. The follistatin containing fraction was optionally buffered to pH 8.5 for solubilizing protein.

Subsequently, the follistatin containing fraction, either as sedimented precipitate or as solubilized protein, was freeze-dried by freezing and applying vaccum to withdraw water, while controlling the temperature of the fraction to preferably not exceed 10°C, preferably 5°C, preferably keeping the fraction in a frozen state.

In the freeze-dried follistatin containing fraction, the content of active follistatin in relation to the total protein concentration was the same as in the liquid egg white after high pressure treatment. This shows that the step of freeze-drying does not substantially affect the activity of follistatin, e.g. freeze-drying does not substantially reduce the concentration of active follistatin per total protein content.

### Example 2: production of a preserved egg white fraction containing active follistatin

Fertilized hen eggs (Gallus domesticus) obtained from a certified breeding station were used, which eggs were not brooded. The eggs were cracked and separated into egg yolk and the white of egg automatically. As the starting material, 3000 L egg white were used that were preferably homogenized by stirring were maintained at 5 to 10°C. Aqueous 12M lactic acid was added to the egg white under stirring until a pH of 5.5 was reached. In an alternative method, the egg white was fermented using added lactic acid bacteria to minimize the content of reducing sugar. In both methods, a precipitate formed that was sedimented by centrifugation at 1000 x g for 5 min.

Prior to preserving, the precipitate was optionally solubilized by raising the pH to 8.5 using buffer, e.g. TrisHCl, NaOH, or carbonate buffer.

In a further alternative, the precipitate was generated by adding KCl to a final concentration of approx. 0.25 M in the egg white, followed by the same centrifugation. Prior to preserving, the precipitate was optionally solubilized by dilution using buffer, e.g. carbonate buffer at pH 8.5.

The precipitate, optionally solubilized, was subjected to high pressure treatment in bags and then freeze-dried using the conditions described in Example 1.

In the alternative, the precipitate, preferably solubilized and diluted in aqueous buffer was subjected to pulsed electric field treatment using the conditions described in Example 3.

### Example 3: Production of freeze-dried egg white containing active follistatin using pulsed electric field treatment

An aliquot of the raw liquid egg white used in Example 1 was treated at a flow rate of 30 L/h at 30°C by pulsed electric field (PEF) of a field strength of 12 kV/cm using unipolar positive pulses having a pulse duration of 10µs at a repetition rate of 200 Hz. At an energy input of 50 to 140 kJ/kg, the viable bacterial contamination was reduced by a factor of 10 and 630 CFU, respectively, as determined by dilution plating.

Using SDS-PAGE, a reduction of active follistatin by approx. 15%, or a residual activity of follistatin of 85% based on the raw egg white was found. No thermal denaturation of the liquid egg white was observed in SDS-PAGE.

A follistatin containing fraction was separated from the PEF-treated egg white by adding aqueous 12 M lactic acid until the mixture was at pH 5.5 white while maintaining about 5°C. The precipitate, which was visible, was sedimented by centrifugation at 1000 x g for 5 min and formed the follistatin containing fraction, the supernatant was discarded. The follistatin containing fraction was optionally buffered to pH 8.5 for solubilizing protein.

Subsequently, the follistatin containing fraction, either as sedimented precipitate or as solubilized protein, was freeze-dried by freezing and applying vaccum to withdraw water, while controlling the temperature of the fraction to preferably not exceed 10°C, preferably 5°C, preferably keeping the fraction in a frozen state.

### Example 4: Concentrating whole egg, white of egg or egg yolk by separation prior to

The process of Examples 1, 2 and 3was repeated with the alteration that before the high pressure treatment egg yolk was the starting material, which was separated by centrifugation at 3343 x g for 20 min into a high density fraction that was collected as a pellet and a low density supernatant fraction. The high density was found the high follistatin fraction.

In the alternative, whole egg or white of egg was separated by centrifugation at 3343 x g for 20 min into a high density fraction that was collected as a pellet and a low density supernatant fraction. Again, the high density was found the high follistatin fraction.

The analysis of the follistatin content is shown below :

| fraction | Follistatin [µg/kg] |
|---|---|
| white of egg, prior to centrifugation | 15 |
| white of egg, pellet | 33 |
| | |
| whole egg, prior to centrifugation | 23 |
| whole egg, pellet | 41 |
| | |
| egg yolk, prior to centrifugation | 4 |
| egg yolk, pellet | 36 |

These results show that the separation of egg yolk, whole egg or egg white to a higher density fraction, corresponding to egg yolk membranes and chalazae, results in an increased concentration of follistatin, which fraction after the step of preservation, preferably with subsequent drying, yields a composition having an increased follistatin concentration.

Optionally, the egg yolk, whole egg or egg white prior to the separation was not homogenized, e.g. the egg yolk, whole egg or egg white was passed through a wide sieve or was stirred to only crack the egg yolk membrane to allow egg yolk to exit, preferably without breaking the egg yolk membrane or chalazae into small pieces. The yolk membrane and/or chalazae could optionally be separated from the starting material concurrent with the sedimentation of the precipitated follistatin containing soluble protein fraction.

From the high density fraction the follistatin containing fraction was concentrated by precipitation and centrifugation, which was then subjected to the step of preservation using the process conditions as described in Examples 1 to 3.

## Claims

1. Process for producing a composition containing biologically active follistatin comprising the step of providing a starting material selected from raw liquid egg yolk, whole egg or egg white originating from avian eggsand/or raw animal blood serum and subjecting at least a fraction of the starting material to a step of preservation while maintaining the temperature at or below 38°C, **characterized by** concentrating a follistatin containing protein fraction from the soluble protein of the starting material by precipitation of a soluble protein fraction containing follistatin and separation of the precipitated fraction and in that subsequent to separation of the follistatin containing fraction from the starting material the follistatin containing fraction is subjected to the step of preservation by subjecting the starting material to a high pressure treatment of at least 4500 bar for at least 1 min, or by first subjecting the starting material to a pulsed electric field treatment of at least 5 kV/cm and then separating the follistatin containing fraction from the preserved starting material by precipitation of a soluble protein fraction containing follistatin and separation of the precipitated fraction, to provide a preserved follistatin containing fraction of the starting material.

2. Process according to claim 1, **characterized in that** the precipition is by acidification and/or addition of water soluble salt and/or addition of a solvent.

3. Process according to claim 2, **characterized in that** the starting material is egg white and the acidification is to pH 6 to pH 5.

4. Process according to claim 3, **characterized in that** the acidification is by fermentation of the starting material while reducing the concentration of sugar by addition of acid producing bacteria.

5. Process according to one of the preceding claims, **characterized in that** the step of preservation consists of high pressure treatment, wherein the follistatin containing fraction subsequent to its separation from the starting material is filled into containers, the containers are sealed and subjected to the high pressure.

6. Process according to one of claims 1 to 4, **characterized in that** the step of preservation consists of subjecting the starting material to a pulsed electric field treatment by an electric field of 5 to 40kV/cm.

7. Process according to one of the preceding claims, **characterized by** drying the preserved starting material, e.g. by freeze-drying or by fluidized bed drying.

8. Process according to claim 7, **characterized in that** between the step of high pressure treatment and the step of freeze-drying, the preserved starting material is transported in the sealed containers in which the raw starting material was subjected to the high pressure treatment.

9. Process according to one of the preceding claims, **characterized in that** the raw liquid egg white is provided by opening fertilized avian eggs and separating the egg yolk from the white of egg.

10. Process according to one of the preceding claims, **characterized in that** the raw starting material prior to the step of preservation is cooled to a maximum temperature of 10°C.

11. Process according to one of the preceding claims, **characterized in that** the raw starting material prior to the step of preservation is degassed.

12. Process according to one of the preceding claims, **characterized in that** the raw liquid egg yolk, whole egg or egg white prior to or concurrent with concentrating to a fraction containing follistatin from the soluble protein of the starting material is concentrated to a membrane containing fraction containing the egg yolk membrane and/or the chalazae.

13. Process according to claim 12, wherein the fraction containing the egg yolk membrane and/or the chalazae is the high density fraction obtained by centrifugation and/or the larger size fraction obtained by size separation.

14. Process according to one of the preceding claims, **characterized in that** the raw liquid egg yolk, whole egg or egg white prior to the step of preservation is diluted with a diluent and then concentrated to a fraction containing the egg yolk membrane and/or the chalazae.

15. Process according to claim 14, wherein the fraction containing the egg yolk membrane and/or the chalazae is the high density fraction obtained by centrifugation and/or the larger size fraction obtained by size separation.

16. Process according to one of the preceding claims, **characterized in that** subsequent to the step of preservation, the preserved starting material is dried and subsequently admixed with a solution of a sugar, a sugar alcohol and/or a sugar polymer and dried for encapsulating the liquid egg yolk, whole egg or egg white.

17. Process according to one of the preceding claims, **characterized in that** subsequent to the step of preservation, the preserved starting material is mixed with preserved egg yolk, whole egg or egg white originating from non-fertilized avian eggs to obtain a pre-determined concentration of follistatin in the mixture.

18. Process according to claim 17, **characterized in that** the preserved starting material is egg white, which is dried prior to mixing with thermally preserved egg yolk, whole egg or egg white originating from non-fertilized avian eggs, which is dried prior to mixing.

19. Process according to one of the preceding claims, **characterized in that** the precipitated follistatin is solubilized from the separated precipitate prior to or following preservation.

## Patentansprüche

1. Verfahren zur Herstellung einer Zusammensetzung, die biologisch aktives Follistatin enthält, das den Schritt des Bereitstellens eines Ausgangsmaterials umfasst, das von Vogeleiern stammendem rohen flüssigen Eigelb, Vollei oder Eiklar und/oder rohem tierischem Blutserum ausgewählt ist und Unterwerfen wenigstens einer Fraktion des Ausgangsmaterials unter einen Konservierungsschritt, während die Temperatur bei oder unterhalb 38 °C gehalten wird, **gekennzeichnet durch** das Konzentrieren einer Follistatin enthaltenden Fraktion aus dem löslichen Protein des Ausgangsmaterials durch Fällung einer löslichen Proteinfraktion, die Follistatin enthält, und Abtrennen der gefällten Fraktion und **dadurch, dass** im Anschluß an die Abtrennung der Follistatin enthaltenden Fraktion von dem Ausgangsmaterial die Follistatin enthaltende Fraktion dem Schritt der Konservierung unterworfen wird durch Unterwerfen des Ausgangsmaterials unter eine Hochdruckbehandlung von wenigstens 4500 bar für wenigstens 1 min, oder durch erst Unterwerfen des Ausgangsmaterials unter eine Behandlung mit gepulsten elektrischen Feldern von wenigstens 5 kV/cm und dann Abtrennen der Follistatin enthaltenden Fraktion von dem konservierten Ausgangsmaterial durch Fällen einer löslichen Proteinfraktion, die Follistatin enthält, und Abtrennen der gefällten Fraktion, um eine konservierte, Follistatin enthaltende Fraktion des Ausgangsmaterials bereitzustellen.

2. Verfahren nach Anspruch 1, **dadurch gekennzeichnet, dass** die Fällung durch Ansäuerung und/oder Zugabe wasserlöslichen Salzes und/oder Zugabe eines Lösungsmittels erfolgt.

3. Verfahren nach Anspruch 2, **dadurch gekennzeichnet, dass** das Ausgangsmaterial Eiklar ist und die Ansäuerung auf pH 6 bis pH 5 erfolgt.

4. Verfahren nach Anspruch 3, **dadurch gekennzeichnet, dass** die Ansäuerung durch Fermentation des Ausgangsmaterials erfolgt, während die Konzentration von Zucker durch Zugabe Säure produzierender Bakterien verringert wird.

5. Verfahren nach einem der voranstehenden Ansprüche, **dadurch gekennzeichnet, dass** der Schritt der Konservierung aus der Hochdruckbehandlung besteht, bei der die Follistatin enthaltende Fraktion im Anschluß an ihre Abtrennung vom Ausgangsmaterial in Behälter gefüllt wird, die Behälter verschlossen und dem Hochdruck unterworfen werden.

6. Verfahren nach einem der Ansprüche 1 bis 4, **dadurch gekennzeichnet, dass** der Schritt der Konservierung aus dem Unterwerfen des Ausgangsmaterials unter die Behandlung mit einem gepulsten elektrischen Feld durch ein gepulstes elektrisches Feld von 5 bis 40 kV/cm besteht.

7. Verfahren nach einem der voranstehenden Ansprüche, **gekennzeichnet durch** das Trocknen des konservierten Ausgangsmaterials, z.B. durch Gefriertrocknen oder Wirbelschichttrocknen.

8. Verfahren nach Anspruch 7, **dadurch gekennzeichnet, dass** zwischen dem Schritt der Hochdruckbehandlung und dem Schritt der Gefriertrocknung das konservierte Ausgangsmaterial in den verschlossenen Containern transportiert wird, in denen das rohe Ausgangsmaterial der Hochdruckbehandlung unterworfen wurde.

9. Verfahren nach einem der voranstehenden Ansprüche, **dadurch gekennzeichnet, dass** das rohe flüssige Eiklar durch das Öffnen befruchteter Vogeleier und Abtrennen des Eigelbs vom Eiklar bereitgestellt wird.

10. Verfahren nach einem der voranstehenden Ansprüche, **dadurch gekennzeichnet, dass** das rohe Ausgangsmaterial vor dem Schritt der Konservierung auf eine maximale Temperatur von 10 °C gekühlt wird.

11. Verfahren nach einem der voranstehenden Ansprüche, **dadurch gekennzeichnet, dass** das rohe Ausgangsmaterial vor dem Schritt der Konservierung entgast wird.

12. Verfahren nach einem der voranstehenden Ansprüche, **dadurch gekennzeichnet, dass** das rohe flüssige Eigelb, Vollei oder Eiklar vor oder gleichzeitig mit dem Konzentrieren zu einer Follistatin enthaltenden Fraktion aus dem löslichen Protein des Ausgangmaterials zu einer Fraktion konzentriert wird, die die Eigelbmembran und/oder die Hagelschnüre enthält.

13. Verfahren nach Anspruch 12, bei dem die die Eigelbmembran und/oder die Hagelschnüre enthaltene Fraktion die Fraktion hoher Dichte ist, die durch Zentrifugation und/oder die durch Größentrennung erhaltene Fraktion größerer Größe ist.

14. Verfahren nach einem der voranstehenden Ansprüche, **dadurch gekennzeichnet, dass** das rohe flüssige Eigelb, Vollei oder Eiklar vor dem Schritt der Konservierung mit einem Verdünnungsmittel verdünnt wird und dann zu einer Fraktion konzentriert wird, die die Eigelbmembran und/oder die Hagelschnüre enthält.

15. Verfahren nach Anspruch 14, bei dem die Fraktion, die die Eigelbmembran und/oder die Hagelschnüre enthält, die Fraktion hoher Dichte ist, die durch Zentrifugation erhalten wird, und/oder die durch Größentrennung erhaltene Fraktion größerer Größe.

16. Verfahren nach einem der voranstehenden Ansprüche, **dadurch gekennzeichnet, dass** im Anschluß an den Schritt der Konservierung das konservierte Ausgangsmaterial getrocknet und anschließend mit einer Lösung eines Zuckers, eines Zuckeralkohols und/oder eines Zuckerpolymers gemischt und getrocknet wird, um das flüssige Eigelb, Vollei oder Eiklar einzukapseln.

17. Verfahren nach einem der voranstehenden Ansprüche, **dadurch gekennzeichnet, dass** das konservierte Ausgangsmaterial im Anschluß an den Schritt der Konservierung mit konserviertem Eigelb, Vollei oder Eiklar gemischt wird, das von unbefruchteten Vogeleiern stammt, um in der Mischung eine vorbestimmte Konzentration an Follistatin zu erreichen.

18. Verfahren nach Anspruch 17, **dadurch gekennzeichnet, dass** das konservierte Ausgangsmaterial Eiklar ist, das vor dem Mischen mit thermisch konserviertem, von unbefruchteten Vogeleiern stammendem Eigelb, Vollei oder Eiklar getrocknet ist, das vor dem Mischen getrocknet ist.

19. Verfahren nach einem der voranstehenden Ansprüche, **dadurch gekennzeichnet, dass** das gefällte Follistatin vor der oder nach der Konservierung von dem abgetrennten Präzipitat solubilisiert wird.

## Revendications

1. Procédé de production d'une composition contenant de la follistatine biologiquement active, comprenant l'étape consistant à fournir un matériau de départ choisi parmi le jaune d'oeuf cru liquide, l'oeuf entier ou le blanc d'oeuf provenant d'oeufs aviaires et/ou de sérum sanguin animal brut et à soumettre au moins une fraction du matériau de départ à une étape de conservation tout en maintenant la température inférieure ou égale à 38°C, **caractérisé par** la concentration d'une fraction de protéine contenant de la follistatine à partir de la protéine soluble du matériau de départ par précipitation d'une fraction de protéine soluble contenant de la follistatine et séparation de la fraction précipitée et en ce que, suite à la séparation de la fraction contenant de la follistatine du matériau de départ, la fraction contenant de la follistatine est soumise à l'étape de conservation en soumettant le matériau de départ à un traitement à haute pression d'au moins 4500 bar pendant au moins 1 min ou d'abord en soumettant le matériau de départ à un traitement de champ électrique pulsé d'au moins 5 kV/cm, puis en séparant la fraction contenant de la follistatine du matériau de départ conservé par précipitation d'une fraction de protéine soluble contenant de la follistatine et une séparation de la fraction précipitée pour fournir une fraction contenant de la follistatine conservée du matériau de départ.

2. Procédé selon la revendication 1, **caractérisé en ce que** la précipitation est par acidification et/ou addition de sel soluble dans l'eau et/ou addition d'un solvant.

3. Procédé selon la revendication 2, **caractérisé en ce que** le matériau de départ est du blanc d'oeuf et l'acidification est comprise entre pH 6 et pH 5.

4. Procédé selon la revendication 3, **caractérisé en ce que** l'acidification est réalisée par fermentation du matériau de départ tout en réduisant la concentration de sucre par addition de bactéries produisant de l'acide.

5. Procédé selon l'une des revendications précédentes, **caractérisé en ce que** l'étape de conservation consiste en un traitement à haute pression, dans lequel la fraction contenant de la follistatine après sa séparation du matériau de départ est versée dans des récipients, les récipients sont scellés et soumis à la haute pression.

6. Procédé selon l'une des revendications 1 à 4, **caractérisé en ce que** l'étape de conservation consiste à soumettre le matériau de départ à un traitement par champ électrique pulsé par un champ électrique de 5 à 40kV/cm.

7. Procédé selon l'une des revendications précédentes, **caractérisé par** le séchage du matériau de départ conservé, par exemple par lyophilisation ou par séchage au lit fluidisé.

8. Procédé selon la revendication 7, **caractérisé en ce que**, entre l'étape de traitement à haute pression et l'étape de lyophilisation, le matériau de départ conservé est transporté dans les récipients scellés dans lesquels le matériau de départ brut a été soumis au traitement à haute pression.

9. Procédé selon l'une des revendications précédentes, **caractérisé en ce que** la fourniture du blanc d'oeuf brut consiste à ouvrir des oeufs aviaires fécondés et à séparer le jaune d'oeuf du blanc d'oeuf.

10. Procédé selon l'une des revendications précédentes, **caractérisé en ce que** le matériau de départ brut avant l'étape de conservation est refroidi à une température maximale de 10°C.

11. Procédé selon l'une des revendications précédentes, **caractérisé en ce que** le matériau de départ brut avant l'étape de conservation est dégazé.

12. Procédé selon l'une quelconque des revendications précédentes, **caractérisé en ce que** le jaune d'oeuf liquide brut, l'oeuf entier ou le blanc d'oeuf avant ou simultanément à la concentration en une fraction contenant de la follistatine à partir de la protéine soluble du matériau de départ est concentré en une fraction contenant des membranes contenant la membrane du jaune d'oeuf et/ou les chalazes.

13. Procédé selon la revendication 12, dans lequel la fraction contenant la membrane de jaune d'oeuf et/ou les chalazes est la fraction de haute densité obtenue par centrifugation et/ou la fraction de plus grande dimension obtenue par séparation selon la taille.

14. Procédé selon l'une des revendications précédentes, **caractérisé en ce que** le jaune d'oeuf liquide brut, l'oeuf entier ou le blanc d'oeuf avant l'étape de conservation est dilué avec un diluant puis concentré à une fraction contenant la membrane de jaune d'oeuf et/ou les chalazes.

15. Procédé selon la revendication 14, dans lequel la fraction contenant la membrane de jaune d'oeuf et/ou la chalaze est la fraction de haute densité obtenue par centrifugation et/ou la fraction de plus grande dimension obtenue par séparation selon la taille.

16. Procédé selon l'une quelconque des revendications précédentes, **caractérisé en ce que**, après l'étape de conservation, le matériau de départ conservée est séchée et ensuite mélangée avec une solution de sucre, un alcool de sucre et/ou un polymère de sucre et séchée pour encapsuler le jaune d'oeuf liquide, l'oeuf entier ou le blanc d'oeuf.

17. Procédé selon l'une quelconque des revendications précédentes, **caractérisé en ce que**, après l'étape de conservation, on mélange le matériau de départ conservée avec de la conserve de jaune d'oeuf, d'oeuf entier ou de blanc d'oeuf provenant d'oeufs aviaires non fécondés pour obtenir une concentration prédéterminée de la follistatine dans le mélange.

18. Procédé selon la revendication 17, **caractérisé en ce que** le matériau de départ conservé est le blanc d'oeuf séché avant mélange avec du jaune d'oeuf conservé thermiquement, des oeufs entiers ou des oeufs provenant d'oeufs aviaires non fécondés séchés avant le mélange.

19. Procédé selon l'une des revendications précédentes, **caractérisé en ce que** la follistatine précipitée est solubilisée à partir du précipité séparé avant ou après la conservation.
